(19) Europäisches Patentamt
European Patent Office
Office européen des brevets

(11) **EP 3 974 808 A1**

(12) **EUROPEAN PATENT APPLICATION**

(43) Date of publication:
**30.03.2022 Bulletin 2022/13**

(21) Application number: **20198427.5**

(22) Date of filing: **25.09.2020**

(51) International Patent Classification (IPC):
**G01N 21/03** [(2006.01)]     *G01N 21/05* [(2006.01)]
*G01N 21/3504* [(2014.01)]     *G01N 21/39* [(2006.01)]

(52) Cooperative Patent Classification (CPC):
**G01N 21/031; H01S 5/0222;** G01N 21/3504;
G01N 2021/052; G01N 2021/399; G01N 2201/0636;
G01N 2201/129; H01S 5/02253; H01S 5/02257;
H01S 5/02407; H01S 5/3401; H01S 5/4087

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **MIRO Analytical AG**
**8304 Wallisellen (CH)**

(72) Inventors:
• **ASEEV, Oleg**
  **8051 Zürich (CH)**
• **LOOSER, Herbert**
  **8049 Zürich (CH)**

(74) Representative: **Clerc, Natalia**
  **Isler & Pedrazzini AG**
  **Giesshübelstrasse 45**
  **Postfach 1772**
  **8027 Zürich (CH)**

(54) **GAS ANALYSER APPARATUS AND METHOD**

(57)    A gas analyser apparatus using laser absorption spectroscopy comprises a multipass cell with a cell body (2), a front mirror holder (3) and a back mirror holder (4). The cell body (2) has a main tube (20) with an interior space for receiving gas to be analysed and a gas inlet (21) for leading the gas into this interior space and a gas outlet (22) for leading the gas out of this interior space. The main tube (20) is made of glass and the gas inlet (21) and the gas outlet (22) are made of glass as well.

FIG. 3

**Description**

TECHNICAL FIELD

The present invention relates to a gas analyser apparatus using laser absorption spectroscopy, preferably mid-infrared (IR) laser spectroscopy, and a method for starting-up a gas analyser apparatus using laser absorption spectroscopy.

PRIOR ART

Today, air pollution and climate change are among the most urgent challenges of mankind. Public air quality monitoring networks have the mission to assess air quality. In addition, polluting industries are obliged to measure and report their emissions, but also have tremendous potential for savings if they can apply online gas analysis in their production processes.

The method of analysing gas concentrations, especially using mid-infrared (IR) laser spectroscopy, is well known in the state of the art. See for example

- "Laser driving and data processing concept for mobile trace gas sensing: Design and implementation", C. Liu et al. RSI 89, 065107 (2018),
- "Multi-species trace gas sensing with dual-wavelength QCLs", M. Hundt et al. APB 124, 108 (2018),
- "High-precision ethanol measurement by mid-IR laser absorption spectroscopy for metrological applications", O. Aseev et al. Optics Express 27, 5314-5325 (2019),
- Appl. Phys. B 90, 165-176 (2008) "Applications of quantum cascade lasers to trace gas sensing" and
- EP 2 940 808 B1.

Gas analyser apparatus are also known in the state of the art. Often, they can measure just one single gas . Therefore, measurement equipment for a multiple of gases is quite bulky, expensive and they cannot be used with moving bases, such as cars, trains or trams. In addition, the known analysers are usually quite complicated in use, so that they can only be operated by well-trained staff.

The applicant has developed and produces a laser based all-in-one analyser for precise and direct measurement of several, even numerous gases, such as the most important pollutants, namely CO, NO, $NO_2$, $NH_3$, $O_3$ and $SO_2$, as well as greenhouse gases such as $CO_2$, $CH_4$, $H_2O$ and $N_2O$, and many more species absorbing in the IR range. The analyser can measure a multiple of gases at the same time. It is easy to handle and reduces the overall costs of such an equipment.

This analyser comprises

- Three lasers in separate housings, namely single mode DFB QCL (Quantum Cascade Laser),
- a multi-pass cell with reduced pressure, the laser light being reflected between two mirrors to create a long interaction path of the light with the sampled gas,
- a photodetector, more precisely an infrared (IR) detector, corresponding to the light source's wavelength (e.g. MCT),
- laser driving electronics,
- means for data-acquisition and AD-conversion and
- a software controlling the system and performing the spectral analysis

The light source (e.g. QCL or ICL (Interband Cascade Laser)) is selected such that its light is absorbed by the target molecules. In case a QCL is used, it is driven in intermittent continuous-wave (iCW) driving scheme with pulse durations between 10-100 $\mu$s and an overall period of about 0.02-1 ms. As the laser rapidly heats up when a driving-current pulse is applied, a tuning in emission frequency occurs. Such tuning is used to record absorption spectra of the desired gas components.

In this analyser, the concentration (i.e. the volume mixing ratio) of each gas is determined by application of Beer-Lambert's law. When light of a mid-infrared laser source is sent through a gas sample containing an absorbing gas species, its absorption can be detected, if the frequency of the light source corresponds to the frequency of a ro-vibrational transition. If the gas temperature, optical path length, pressure and absorption cross section are known, the gas-concentration (mixing ratio) can be determined by an analytical fitting of the individual absorption lines.

In "Mid-IR spectrometer for mobile, real-time urban NO2 measurements", M. Hundt et al. AMT 11, 2669-2681 (2018), it was proofed that such an analyser comprising one laser can even be used for mobile measurements. It was actually mounted on the roof of a tram travelling for months through the city of Zurich.

The existing analyser of the applicant performs at a high quality level. However, there is always room for improvement.

SUMMARY OF THE INVENTION

It is therefore an object of the invention to provide a gas analyser having an improved measurement accuracy, precision, reliability and user-friendliness.

This object is solved by a gas analyser using laser absorption spectroscopy with the features of claim 1.

The inventive gas analyser using laser absorption spectroscopy comprises a multipass cell with a cell body, a front mirror holder and a back mirror holder, wherein the cell body has a main tube with an interior space for receiving gas to be analysed and wherein the cell body has a gas inlet for leading the gas into this interior space and a gas outlet for leading the gas out of this interior space. The main tube is made of glass and the gas inlet and the gas outlet are made of glass as well.

Multipass cells in the state of the art are sometimes made of metal, for example steel or aluminium. Most of of the multipass cells comprise a glass tube combined with a metal gas-inlet and a metal gas-outlet and metal mirror-holders.

It has been found that especially some gases cannot be measured with sufficient accuracy. It has also been found that metal gas inlets and outlets lead to adsorption of "sticky" or reactive molecules such as $NO_2$, $NH_3$, or $H_2O$, which slows down the gas throughput of the cell and therefore increases the time-response of the instrument or even alters the measurement result due to chemical reactions taking place on the metal surface.

The inventive analyser comprises a multipass cell with a cell body made of glass with a gas inlet and a gas outlet made of glass. Preferably, the cell body is made by glassblowing, thereby attaching the pre-manufactured gas inlet and gas outlet to the main glass tube of the cell body by welding. Preferably, the cell body consists completely of glass. In preferred embodiments, Pyrex glass is used because of its low thermal expansion.

Since the inlet and the outlet is made of glass, the interaction of the sampled gas with metal surfaces is reduced to a minimum and therefore the instruments rise- and fall-time is reduced allowing fast sampling.

Preferably, the gas inlet and the gas outlet are shaped as tubes. Preferably, they have the shape of a circular cylinder. Therefore, connecting tubes leading to an inlet and an outlet of the gas analyser apparatus can be easily mounted. Preferably, the tubes are made of PTFE (polytetrafluoroethylene) or PFA (perfluoralkoxy alkane) or another appropriate material.

This overall arrangement of a multipass cell body, inlet and outlet made completely of glass also reduces the productions costs.

In preferred embodiments, the mirror-holders are made of aluminium.

The analyser apparatus comprising the inventive multipass cell can comprise one or more lasers used for measurement.

In this text, lasers are mentioned. Lasers are herein understood to be the laser emitting light for measurement. The apparatus may comprise additional lasers, especially for alignment reasons, for example visible red lasers.

The accuracy and the reproducibility of gas mixing ratios as well as the user-friendliness of the analyzer can be improved, when the instrument's light sources used for the measurement, usually the lasers, are tuned to exactly the same ro-vibrational absorption lines at each startup and when they are kept exactly at this emission frequency (wavelength) during the measurement. In addition, it is advantageous if the instrument has the ability to find the correct spectral features in case the fit was lost.

Depending on environmental parameters, such as temperature, and on drifts of the laser devices, the laser-temperature that is required to emit at a given frequency can vary. The state of the art analysers try to control the laser temperature. However, this is not sufficient to reproduce the exact same absorption spectrum from one instrument startup to another, since the laser frequency is usually not exactly the same as during previous measurements and therefore, the position of the absorption lines of interest in the spectrum may change.

After a shutdown and restart, the spectrum may be shifted and the amplitude may be reduced or increased.

It is therefore an object of the invention to provide a method, which enables an optimized but quite short start-up of the gas analyser.

This object is achieved with a method according to claim 13.

When setting up an instrument with a new laser or restarting the instrument after a shutdown, the absolute position of the tuning range of the laser might be known only vaguely. In addition, the tuning itself as well as the dependence of the tuning range on the laser temperature is substantially nonlinear. In order to determine the targeted spectral range spectral fingerprinting, using for example the HITRAN database, is thus performed (see Journal of Quantitative Spectrocopy and Radiative Transfer, Volume 203, December 2017, Pages 3-69, Elsevier).

The inventive method profits from two experimental observations/facts:

- The relative tuning of the laser as a function of time can be determined using an etalon. In most cases, the laser temperature dependence of the tuning, which is usually nonlinear, is small within typical absorption line position deviation and thus a single determination of the relative tuning is sufficient for performing the fingerprinting.

- The temperature dependence of the "shape" of the power output of the laser as a function of time (P(t)) / current (P(I)) is preferably small. However, the absolute power, detected by the photodiode, might vary significantly with temperature and is sometimes significantly different after a shutdown/restart of the instrument due to up to now not fully understood reasons. In other cases, vibrations invoke small misalignment of the optics that also results in a change of the absolute power detected but not its shape as a function of time.

The inventive algorithm provides sufficiently good starting values for the nonlinear regression, with which the "strength" of the absorption lines and finally the concentration of the according gases in the measurement cell is determined.

The inventive method comprises the steps of

1. Make a good guess for the "bare laser power" (herein called P*(t), wherein t stands for time)
A least squares regression of a "bare laser power"- shape to the experimental data will always cross the absorption lines and turned out to be in general not of sufficient quality.
However, the signal to noise ratio is large enough that a function,

$$P^*(t) = a \cdot P_{Setup}(t)$$
$$= \left(1 - \frac{Min\left(P_{Setup}(t) - f_{exp}(t)\right)}{P_{Setup}}\right) P_{Setup}(t)$$

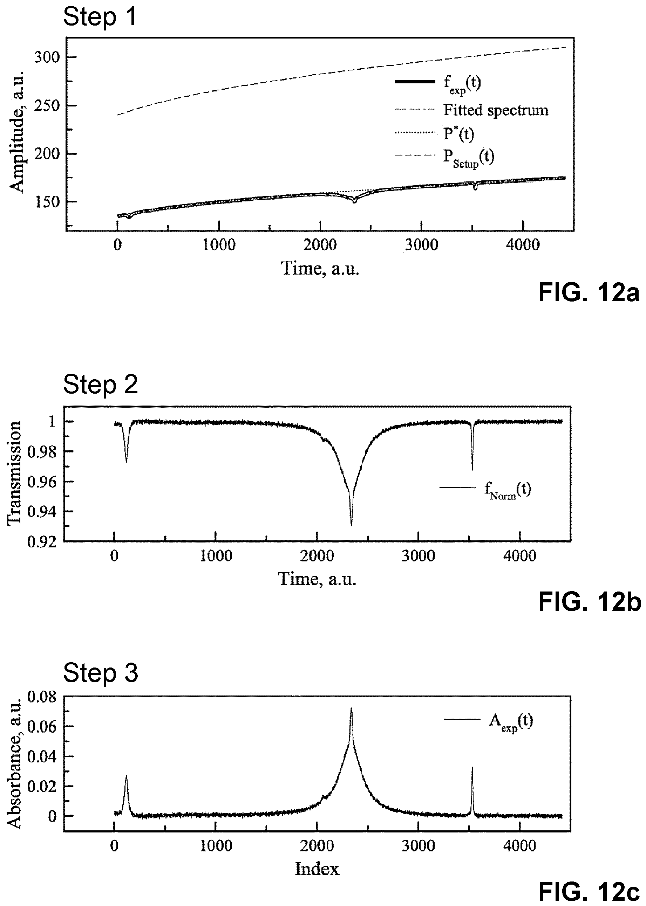

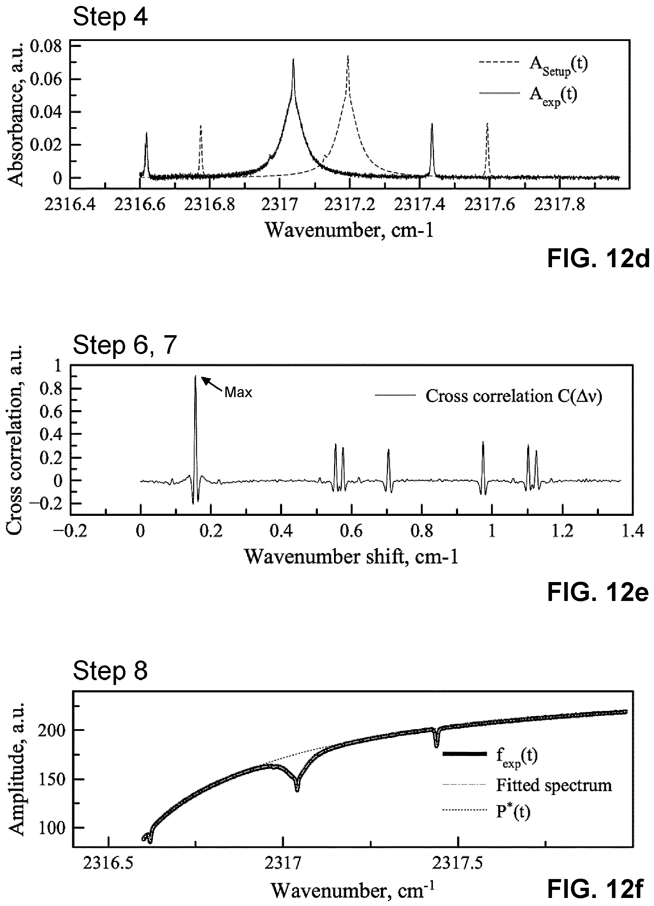

that "touches" the experimental data from above, is a good approximation.
$P_{Setup}(t)$ denotes the "former bare laser power" determined in a previous Setup-procedure or by some other means.
$f_{exp}(t)$ is the experimental spectral data measured in this measuring session and it is also called the detected spectrum.
Min is the minimum of the difference of $P_{Setup}(t)$ and $f_{exp}(t)$. It follows:
$P^*(t) \approx 2/3 \cdot P_{Setup}(t)$ in the particular example shown in figure 12a (step 1).

$$f_{Norm}(t) = \frac{f_{exp}(t)}{P^*(t)}$$

2. Normalize detected spectrum (transmission spectrum):
For the normalization of the experimental spectrum $f_{Norm}(t)$, $f_{exp}(t)$ is divided by the $P^*(t)$.

3. Calculate the experimental absorbance $A_{exp}(t) = -ln(f_{Norm}(t))$
The transmission $f_{Norm}(t)$ decreases exponentially with increasing concentration.
Therefore, this nonlinearity would falsify the succeeding cross correlation and is thus fixed by taking the logarithm (i.e. ln).

4. Transform the experimental absorbance $A_{exp}(t)$ into a "frequency-equi-spaced" absorbance $A(v)$, where v is the frequency of the laser light, using the according tuning curve.
In order to identify the "spectral finger print" by cross correlation and thus obtain the absolute frequency position of the spectral window investigated, the data points of $f_{exp}(t)$ must be equispaced in frequency space.

5. Differentiate the absorbance $A(v)$: $A'(v) = dA/dv$
Changes of the shape of the "bare laser power" P*(t) can result in significant "ghost signal amplitudes". As, however, the "bare laser power" is of much lower frequency content than the absorption lines, using the derivative increases the robustness of the algorithm substantially. According to the invention claimed the derivative $A'(v)$ and $A'_{Hitran}(v)$ are correlated. $A'_{Hitran}(v)$ denotes the derivative of simulated absorbance based on the HITRAN Database or on a custom made database, for example in case if the species are not included in HITRAN.
This is a procedure used in edge detection/pattern recognition, but this step is new in this context. The noise in the derivative is significantly increased. However, this noise is reduced afterwards by the cross correlation, as the theoretically calculated absorbance $A_{Hitran}(v)$, with which the experimental data are correlated, is noise free.

6. Perform the cross correlation C(v):

$$C(\nu) = A'(\nu) \star A'_{Hitran}(\nu)$$

7. Locate the maximum Max($C(v)$) in the cross correlation to obtain the absolute frequencies $v_{absolute}$(t) of experimental data set of the detected spectrum $f_{exp}$(t).

8. Perform a nonlinear regression on $f_{exp}$(t), based on the Hitran data-based caculated spectra or based on a custom made database, for example in case if the species are not included in HITRAN. In this fit only the "bare laser power"-parameters as well as the concentrations (intensity of absorption lines) are fitted whereas the absolute frequencies $v_{absolute}$(t) are kept fix, set to the positions determined in correlation step 7. The data from the custom made database used are preferably generated under the same or similar experimental conditions.

9. Use the found absolute frequencies $v_{absolute}$(t), concentrations and parameters of the "bare laser power" P*(t) as starting parameters for further fitting. By this the nonlinear regression, with which the transmission of the absorption lines and finally the concentration of the according gases in the measurement cell is determined.

Figures 12a to 12f show graphs illustrating the start-up algorithm.

As the range of the theoretically calculated pattern is only limited by computer memory, the above described procedure serves both as restart function of an instrument after a shutdown, where the target absorption lines measured are still within the experimentally scanned frequency range as well as for characterisation of a new laser, where the frequency range covered by the laser is much more uncertain, i.e. where real "finger printing" is needed.

This starting-up algorithm is preferably used in the gas analyser apparatus of the invention claimed herein. However, it can also be used in the already known gas analyser apparatus of the applicant or in other kind of gas analyser apparatus, especially in analysers using direct laser absorption spectroscopy.

In addition, it is quite difficult to align analysers using direct laser absorption spectroscopy and a multi-pass cell of the astigmatic Herriott type, since they need careful adjustment of mirror rotations, Z-positions and tilt-angles.

It is another object of the invention to provide a gas analyser using laser absorption spectroscopy, especially an analyser using an astigmatic Herriott cell, which can be assembled and aligned in a quite simple way.

This object is solved by a gas analyser using laser absorption spectroscopy with the features of claim 14.

The inventive gas analyser apparatus using laser absorption spectroscopy comprises an optical sample cell, such as a longpath cell or a multipass cell including a front mirror holder and a back mirror holder. The back mirror holder comprises a back mirror and adjustment means for adjusting the back mirror. The adjustment means comprises a lever arm which is rotatable around a longitudinal middle axis of the optical sample cell by movement of a micro-screw which can be moved in a direction perpendicular to the longitudinal middle axis of the optical sample cell.

The inventive mirror holder has therefore, in addition to the typical mirror-mount adjustments enabling mirror tilt angles in the XOZ and YOZ planes, a mechanical construction for precise control of the mirror rotation around Z axis and translation along Z axis. Since the construction comprises of a spring-held lever arm that can be pushed precisely by a micro-screw, adjustement is simplified. Especially, the mirror can be adjusted precisely and independently with regard to four degrees of freedom.

This inventive mirror holder is preferably used in the gas analyser apparatus of the invention claimed herein, especially in combination with a multipass cell as mentioned above and/or a laser housing as mentioned below. However, it can also be used in the already known gas analyser apparatus of the applicant or in other kind of gas analyser apparatus, especially on analysers containing a multipass cell of the astigmatic Herriott type.

As mentioned in the introductory part, most laser-based gas analyzers contain only one laser device. In those cases where several laser devices are used, the lasers are packaged in individual housings or laser-modules. Having several single laser devices packaged individually results in bulky optical setups and increase effort for alignment of the lasers.

It is another object of the invention to provide a gas analyser using laser absorption spectroscopy and comprising at least two lasers, wherein the gas analyser can be assembled and adjusted in a quite simple way.

This object is solved by a gas analyser using laser absorption spectroscopy with the features of claim 15.

The inventive gas analyser apparatus using laser absorption spectroscopy comprises at least two lasers, preferably up to five, six, seven or even more lasers, and a laser housing. The at least two lasers are mounted in the laser housing comprising a monolithic main body defining an interior space for receiving the lasers and a lid for sealingly closing the main body. The laser housing is arranged within an outer housing of the gas analyser apparatus.

The inventive gas analyser comprises a laser housing that can accommodate at least two lasers, preferably QCL (Quantum Cascade Laser) or ICL (Interband Cascade Laser), or TDLs (tuneable diode lasers, or even dual-color QCL. In preferred embodiments, the laser housing can accommodate up to five QCL or ICL chips. In other embodiments, the laser housing can accommodate even more than five lasers, such as six, seven or even more.

The housing preferably contains two, three, four, five or more slots, which are preferably defined by threaded holes in the bottom of the housing, where lasers are placed.

The laser housing is preferably made of a monolithic main body, which is closed with a lid. The lid is preferably arranged on the top of the main body. The main body is preferably milled from one single block. This makes the setup rugged against shock and vibration

The block and/or the lid are preferably made of aluminium. The main body is preferably hermetically sealed at this top with the lid and a sealing element, such as an O-ring.

In preferred embodiments, the main body or the lid comprises a port for filling the interior of the housing with an inert gas, for example with Argon. This ensures to remove water vapour and thus eliminates its condensation on the lasers, which can damage them.

In preferred embodiments, the port can be used to evacuate the interior of the housing prior to fill it with the inert gas.

In preferred embodiments, their main body comprises windows in the shape of through-openings arranged in one sidewall of the main body. The light of the lasers leaves the housing through these windows, wherein each window is assigned to one laser. A collimating or focusing optic, such as at least one lens is arranged within each through-opening or shortly before or after each window, seen in the direction of the light path. Preferably, the at least one lens is arranged outside in front of each window.

Preferably, the positions of the lenses can be adjusted in x-y-z directions. This makes the optical alignment easy. Since the lenses are mounted to the main body, they can be coaligned with the lasers before these devices are arranged within the outer housing of the analyser apparatus. In case visible lasers, such as red lasers, are used, they can be arranged in the laser housing as well, which helps the alignment process. The visible and the measuring lasers, which are usually IR lasers, can be coaligned in the laser housing before placing the lasers in the analyser housing. This makes assembly of the whole apparatus easier and therefore reduces production costs. In addition, the setup is even better rugged against shock and vibration and makes it easier to replace lasers in a case of servicing, since the entire laser housing can be replaced as a whole.

In preferred embodiments, the individual lasers are placed on heat-sinks within the main body. The heat-sinks are preferably made of copper and they are preferably temperature controlled. The temperature control may be established by Peltier elements. Wires of the different elements used, such as wires for temperature sensors, Peltier elements and the lasers preferably enter the housing by air-tight feed-throughs. Depending on the embodiments, the feed-throughs are arranged in the lid. In other embodiments, they can also be arranged in the main body.

This inventive laser housing is preferably used in the gas analyser apparatus of the invention claimed herein, especially in combination with a multipass cell and/or a mirror holder as mentioned above. However, it can also be used in the already known gas analyser apparatus of the applicant or in other kind of gas analyser apparatus, especially on analysers using a multi-pass cell of the astigmatic Herriott type.

Further embodiments of the invention are laid down in the dependent claims.

BRIEF DESCRIPTION OF THE DRAWINGS

A preferred embodiment of the invention is described in the following with reference to the drawings, which are for the purpose of illustrating a present preferred embodiment of the invention and not for the purpose of limiting the same. In the drawings,

Figure 1            shows a schematic view of a gas analyser apparatus according to the invention;

Figure 2            shows a schematic view of a gas analyser system with the inner elements of gas analyser according to figure 1;

Figure 3            shows perspective view of a multipass cell according to the invention;

Figure 4            shows a side view of the multipass cell according to figure 3;

Figure 5            shows an exploded view of a part of the multipass cell according to figure 3;

Figure 6            shows another exploded view of a part of the multipass cell according to figure 3;

Figure 7            shows a part of the multipass cell according to figure 3;

Figure 8            shows a longitudinal sectional view of a part of the multipass cell according to figure 3, the section being taken azentrically;

Figure 9            shows an exploded view of a laser housing according to the invention;

Figure 10      shows a partial longitudinal sectional view of the laser housing according to figure 9;

Figure 11      shows a cross sectional view of the laser housing according to figure 9;

Figure 12a to Figure 12f      graphs showing the steps in the start-up slgorithm and

Figure 13      a flow chart of the start-up algorithm.

DESCRIPTION OF PREFERRED EMBODIMENTS

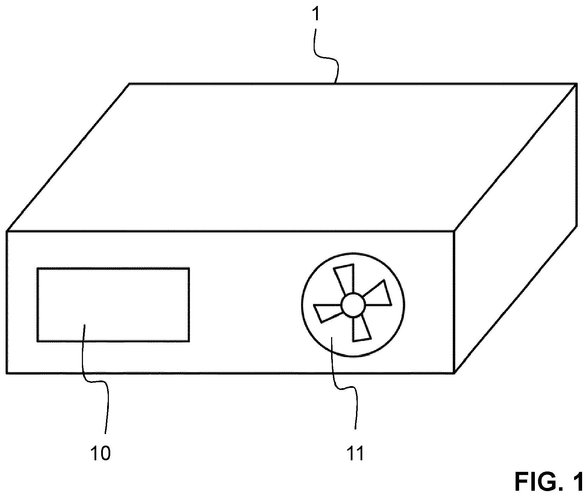

Figure 1 shows a schematic view of a gas analyser apparatus according to the invention. Preferably it is based on the apparatus of the applicant which is already on the market, preferably comprising the components of this apparatus where applicable and where not replaced by the inventive parts and methods.

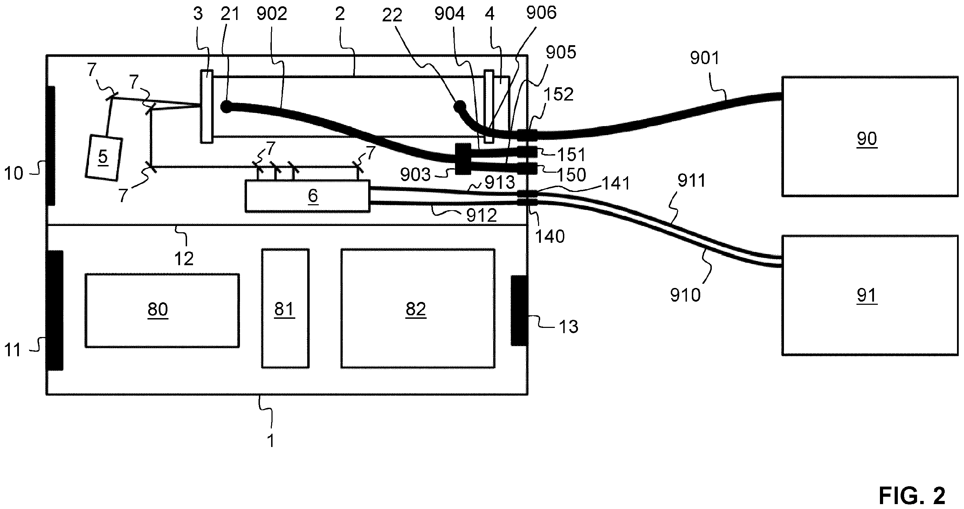

The apparatus comprises an outer housing 1 with at least one fan 11 and a display 10. Depending on the embodiment, the display 10 can be a touch screen as well and act as a machine user interface. On the rear side, which cannot be seen in figure 1 but in figure 2, there is an electronic interface 13 with plug connections and a power switch, a first coolant port 140, a second coolant port 141, a first port 150, a second port 151 and a third port 152.

As can be seen in figure 2, the inventive gas analyser preferably comprises two sections in one single housing 1, wherein these two sections are preferably divided by a dividing wall 12 or an internal enclosure.

The first section forms the optical part, the second section forms the electronics part.

The optical part comprises a multipass cell with a cell body 2, a front mirror holder 3 and a back mirror holder 4, a photodetector 5, a laser housing 6 and deflection mirrors 7. The light path is marked in figure 2 with the reference sign L. These optical elements are preferably mounted on one single base plate, which is preferably fixed to the bottom of the housing in a shock absorbing way. The single base plate is preferably made of carbon-fibre or aluminum. It is not shown in the figures.

This optical part of the gas analyser apparatus is connectable to an outside suction pump 90, preferably a membrane vacuum pump, and a cooling device 91, preferably a water chiller. The gas analyser apparatus, the pump 90 and the cooling device 91 form a gas analyser system. Additional parts can be present in this system as well.

A first external coolant line 910 leads a cooled or warmed fluid, preferably water or another liquid cooling agent, to the first coolant port 140. A first internal coolant line 912 guides the cooled fluid to the laser housing 6, a second coolant line 913 leads from the laser housing 6 to the second coolant port 141 and through a second external coolant line 911 to the cooling device 91. The fluid is usually lead through the base of the monolithic laser housing by dedicated tunnels. The cooling device 91 is preferably used to cool other parts and elements of the gas analyser apparatus as well.

The multipass cell is preferably an astigmatic multipass cell of the Herriott type, preferably with a range of several meters to several tens or even hundreds of meters absorption path.

The cell body 2 of the multipass cell comprises a gas inlet 21 and a gas outlet 22. The gas inlet 21 is connected by an inlet line 902 to a valve 903. The valve 903 is preferably a three-way valve, for example a solenoid valve. The valve 903 can switch between a first gas line 904 and a flushing line 905, the two lines being connected to the second port 151 and the first port 150 respectively.

The gas outlet 22 of the cell body 2 is connected by a second gas line 906 to the third port 152, which lead through an external suction line 901 to the suction pump 90.

A gas sample to be analysed enters the apparatus through the second port 151 and is sucked by the suction pump 90 through the multipass cell. Preferably, the measuring rate is chosen such that it is either comparable or greater than the multipass cell gas exchange rate.

The gas can be a mixed gas and several components of the mixed gas can be measured.

By switching the valve 903, the cell body can be filled with a reference gas with known concentrations or a non-absorbing "zero"-gas (e.g. nitrogen) for automatic calibration and offset corrections.

The laser housing 6 comprises at least one, preferably at least two lasers. Laser light emitted from the lasers are guided by the deflection mirrors 7 through a hole in the front mirror arranged in the front mirror holder 3 into the cell body 2. The laser light is multi-passing the cell body 2 a multiple of times before it leaves the multipass cell through the hole of the front mirror again. It is then detected by the photodetector 5. The detector is preferably a photovoltaic MCT-based or InAsSb-based infrared detector.

At least the photodetector 5 and the lasers of the laser housing 6 are connected with the electronic part of the apparatus. This electronic part preferably comprises a data acquisition device (DAQ) 80, a data processing device 81 and driving electronics 82. Preferably, the suction pump 90 and the cooling device 91 are also controlled by the electronic part of the apparatus or they receive and/or send at least data from respectively to each other. The electronic part preferably comprises active temperature control elements for each of the lasers. The DAQ is preferably a FPGA based data-acquisition system to record the absorption signals detected by the detector 5. The data processing device 81 processes, controls, stores and displays data.

In general and with some exceptions, these main parts of the gas analyser system are known in the state of the art.

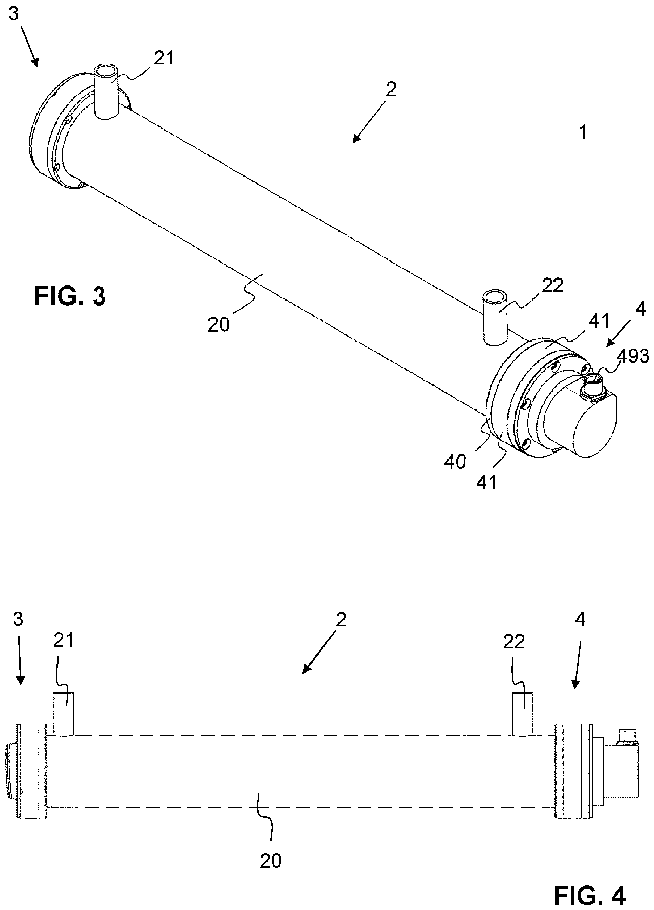

However, the cell body 2 according to the inventive apparatus comprises a main tube 20 made of glass and the gas inlet 21 and gas outlet 22 are made of glass as well (see figure 3). The main glass tube 20 defines an interior space for receiving gas to be analysed. Preferably, it has the shape of an elongate circular cylinder. The gas inlet 21 and the gas outlet 22 have the shape of tubes, preferably also in the form of circular cylinders. Preferably, they are arranged on the lateral surface of the main glass tube 20 near the front and the back mirrors of the cell. Preferably, they extend perpendicular to the longitudinal axis of the main glass tube 20. Preferably, the inlet and the outlet both extend in the same direction. In other embodiments, they extend perpendicular to each other or at an angel to each other.

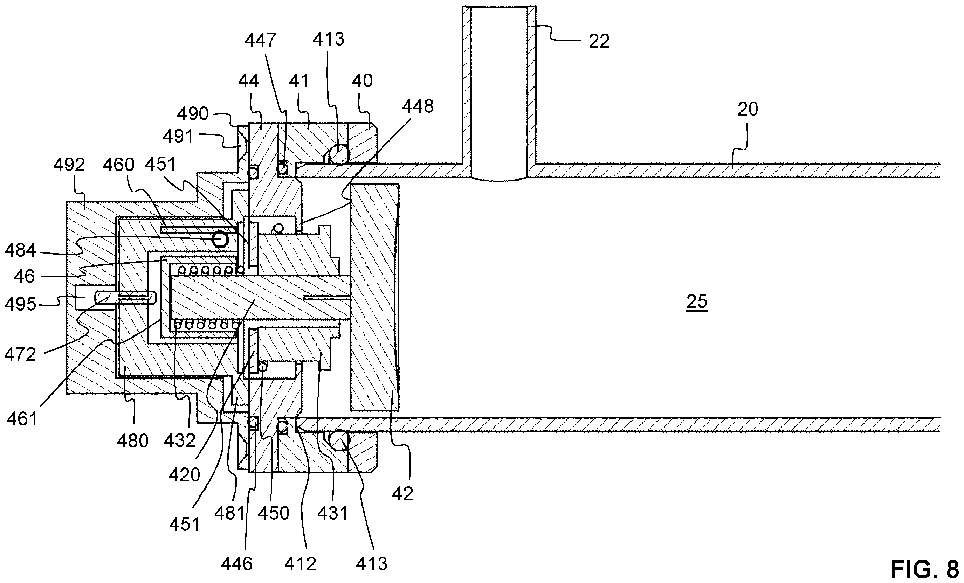

As can be seen in figure 8, the gas outlet 22 is directly fixed to the main glass tube 20 so that this part of the cell body 2 only comprises glass and no other material, even not some adhesive. The same applies for the gas inlet 21 and the region of the main glass tube 20 around the gas outlet 22. Preferably, the main glass tube 20, the gas inlet 21 and the gas outlet 22 are produced of glass as single tools and then gas inlet 21 and the gas outlet 22 is fixed to the main glass tube 20 by glass blowing.

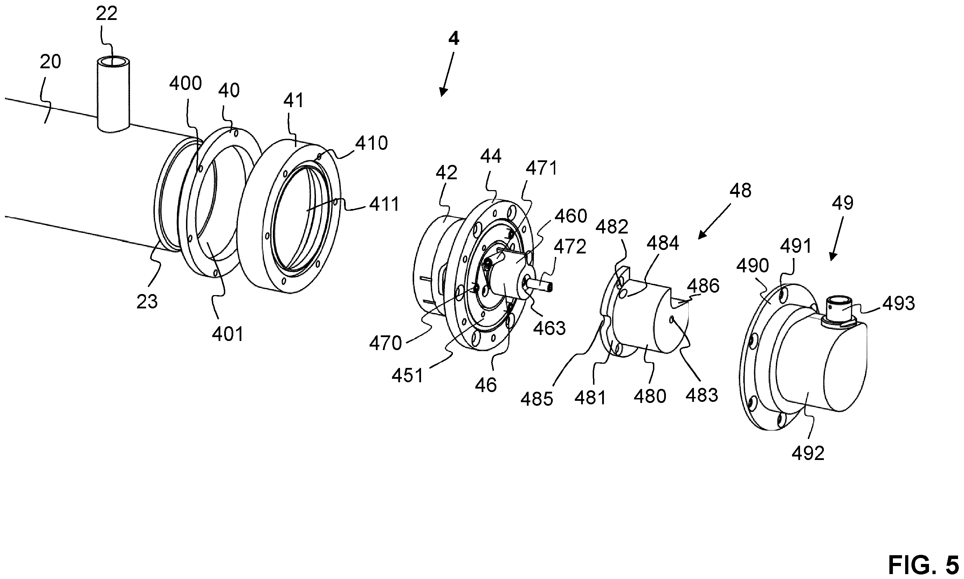

The front face and the back face 23 of the main glass tube 20 is preferably plane and comprises not threads or anything else, as can be seen in figures 5 and 8.

The gas inlet 21 and the gas outlet 22 are preferably arranged at a distance to the front face and the back face 23 of the main glass tube.

The inventive multipath cell comprises a minimum of metal. Preferably, only the mirrors are coated with metal and the mirror holders are at least partially made of metal. Preferably, the metal surface of the holders are located on the rear side of the mirrors, so that sampling effect is improved in case of a constant flush through the cell.

The front mirror holder 3 is not shown in detail. It comprises at least one mounting ring, preferably made of aluminium with a window, and a front mirror, which has a hole for the laser beams to enter and exit the cell. The front mirror holder 3 is preferably shaped as known in the state of the art and does therefore not have to be described in detail. Preferably, for the sake of simplicity the front mirror is preferably fixed and not adjustable.

Figures 5 to 8 show the special back mirror holder 4 of the inventive gas analyser apparatus. Figure 5 shows on its left hand side the rear end part of the main glass tube 20 with the back face 23 and the gas outlet 22. A first mounting ring 40 is next to the back face 23. The first mounting ring 40 comprises a central through-opening 401 with a diameter, which is almost the same as or which is larger than the outside diameter of the main glass tube 20. As can be seen in figure 8, the first mounting ring 40 encompasses the rear end of the main glass tube 20.

The first mounting ring 40 comprises fixation openings 400 for fixing to first mounting ring 40 to a second mounting ring 41. A third mounting ring 44 is screwed to the other side of the second mounting ring 41. An endpiece 49 ist screwed to the third mounting ring 44.

The second mounting ring 41 follows the first mounting ring 40, the second mounting ring 41 preferably being thicker than the first mounting ring 40. It comprises a central through-opening 411 and fixation openings on both sides. The fixation openings 410 fixation of the third mounting ring 44 are shown in figure 5.

As can be seen in figure 8, the second mounting ring 41 comprises a circumferential inner step 412, which is abutted by the rear end face 23 of the main glass tube 20. A sealing ring 413 is arranged between the first and the second mounting ring 40, 41. The glass tube is sealed by these O-rings when the ring 40 presses the ring 41. In order to fix the rings to glass tube edges, the glass tube is evacuated. In other embodiments, bars are used extending along the glass tube and fixing the front and the back mirror holder.

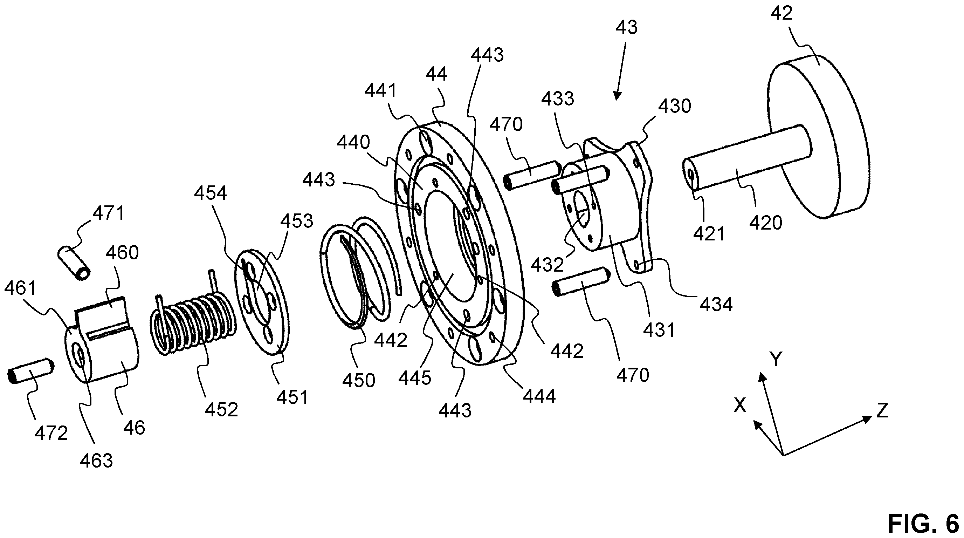

The next part comprises several items, which are shown in more detail in figure 6. It forms a basic mirror holder with the adjustment means, especially some micro-screws 470, 471, 472 and a lever arm 460, and the mounting ring 44 , which basic mirror holder mounts the back mirror 42 to the cell body 2.

In figure 6, the direction of view is changed. This means that elements being on the left side in figure 5 are now arranged on the right side in figure 6.

This part comprises a back mirror 42, which is preferably highly reflective for the wavelengths provided by the lasers. The mirror 42 is fixed to a stem 420 having a central opening 421. A lever arm body 46, which will be described later in the text, is screwed to this stem 420 by using this opening 421. The opening for receiving the fixation screw needed for fixing the lever arm body 46 to the stem 420 has the reference number 463.

The stem 420 penetrates a guiding element 43, which holds and guides the stem 420. The guiding element 43 comprises a main body 431 with a central through-opening 432 for receiving the stem 420 and with a flange 430 directed to the stem 420. The flange 430 has some fixation openings 433 arranged on its circumference. The flange 430 comprises three arms extending radially outside. Each arm comprises an indentation 434 for receiving the free ends of a first micro-screw 470. Preferably, there are three first micro-screws 470. Individual pressure can be applied on each arm of the flange 430, which then can tilt the stem 420 and which can therefore tilt the mirror 42 along the X and Y axes.

The main body 431 of the guiding element 43 penetrates a central through-opening 445 of a middle ring 440 being arranged in a third mounting ring 44. The middle ring 440 and the third mounting ring 44 are made of one single piece. The circumferential rim seen in figure 6 is used for holding a seal ring 446 i.e. an O-ring, as can be seen in figure 8. On the other side of the third mounting ring 44, a further sealing ring 447

The third mounting ring 44 comprises outer fixation through-openings 441 matching with the fixation openings 410 of the second ring 41 so that the third mounting ring 44 can be fixed with screws to the second mounting ring 41 and to the main glass tube 20. The third mounting ring 44 also comprises outer fixation openings 444 in the form of threaded holes for fixing a cap 49 described later in the text. The middle ring 440 comprises inner fixation openings 442 and micro-screw openings 443 for fixinig the microscrew holder 48.

An inner ring 451 is arranged within the central through-opening 445 of the middle ring 443. It is fixed to the main body 431 of the guiding element 43. There are fixation through-openings 454 which corresponds to the fixation openings 433 of the guiding element 43. The screws needed for these fixations are not shown in the figures, as they are not shown for all the other fixations openings mentioned in this text. The inner ring 451 comprises a central through-opening 453 as well. This opening is penetrated by the stem of the back mirror 42, as can be seen in figure 8.

A first spring 450 is arranged between the inner ring 451 and an inner flange 448 of the middle ring 440 being part of third mounting ring 44. The first spring 450 is arranged around the main body 431 of the guiding element 43, as can be seen in figures 6 and 8. The first micro-screws 470 extend through the body of the middle ring 440, which cannot be seen in the sectional view of figure 8. The first spring 450 pushes the guiding element 43 towards the first micro-screws 470.

On the opposite side of the inner ring 451, a lever arm body 46 with a protruding lever arm 460 is arranged. The lever arm body 46 has a cylindrical shape and comprises a blind hole into which the stem 420 extends. This can be seen in figures 6 and 8. Within the blind hole of the lever arm body 46, the stem 420 is surrounded by a second spring 452. The second spring 452 has two functions. It pushes the mirror assembly towards the third micro-screw 472 and it provides a rotational force such that the lever arm pushes the second micro-screw 471.

As can be seen in figure 6, the inner ring 451 comprises a slit 455 for fixing the second spring 452 preventing it from free rotation. A rotational movement of the lever arm body 46 around the longitudinal axis of the sample cell leads to a rotational movement of the stem 420 of the mirror 42. The rotational movement of the lever arm body 46 is caused by a second micro-screw 471 pushing on the lever arm 460 in a direction perpendicular to the longitudinal axis of the cell body 2. It is used for the z-axis rotation adjustment of the back mirror 42.

The translation adjustment of the back mirror 42 along the longitudinal axis of the cell body 2, i.e. the z-axis, is performed by movement of a third micro-screw 472 which acts on a front face 461 of the lever arm body 46. Like this, the back mirror 43 can be adjusted by translation and rotational movement with independent adjustment means, i.e. the micro-screws 470, 471, 472. Especially the z-rotational adjustment and the translation along the z-axis can be made independently of the x,y rotational adjustment.

Going back to figure 5, the assembled part forming the basic mirror holder shown in figure 6 in an explosive view can now be better understood. This assembled basic mirror holder can be attached and fixed as one single piece to the second mounting ring 41 and therefore to the cell body 2. As can be seen in figures 5 and 8, the lever arm body 46 is encompassed by a micro-screw holder 48. The micro-screw holder 48 comprises a main body 480 with a cylindrical shape and having a hole. A flange 481 with fixation through openings 482 surrounds the hole.

A central micro-screw opening 483 extends along the longitudinal axis into the blind hole and a lateral micro-screw opening 484 extends in radial direction into the main body 480 of the micro-screw holder. The micro-screw holder 48 holds the second and the third micro-screw 471, 472.

The three first micro-screws 470 are located within some indentations 485 of the flange 481 of the micro-screw holder 48 and are independent of this holder 48.

A recess 486 in the main body 480 of the micro-screw holder 48 enables the lever arm 460 to be moved.

The invented back mirror holder 4 comprises an end piece 49. It has a main body 492 with a cylindrical shape and a blind hole. A sensor port 493 in the form of an electrical feedthrough allows measurement of the temperature inside the cell.

A flange 490 comprising fixation through openings 491 is directed towards the cell body 2. The flange 490 can be fixed to the third mounting ring 44, since the fixation through openings 491 correspond to the outer fixation openings 444 of the third mounting ring 44. The blind hole has a step. The part with the greater inner diameter encompasses the micro-screw holder 48, the part with the smaller inner diameter accommodates the central micro-screw 483. This arrangement allows to reduce the dead volume where gas could be trapped.

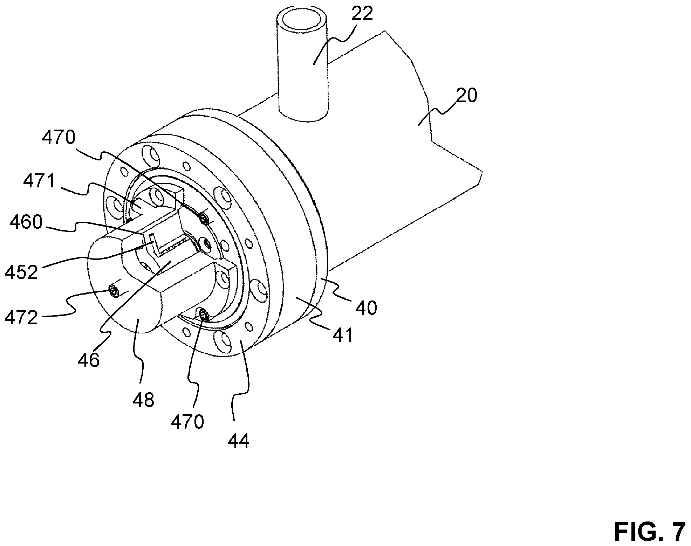

As can be seen in figure 5, the back mirror holder 4 can be easily mounted and disassembled for cleaning. Readjustment is quite easy to handle. In case a readjustment is necessary before or during operation, the end piece 49 can easily be removed and all the micro-screws 470, 471, 472 necessary for the adjustment are readily accessible. Figure 7 shows the situation when the end piece 49 is removed.

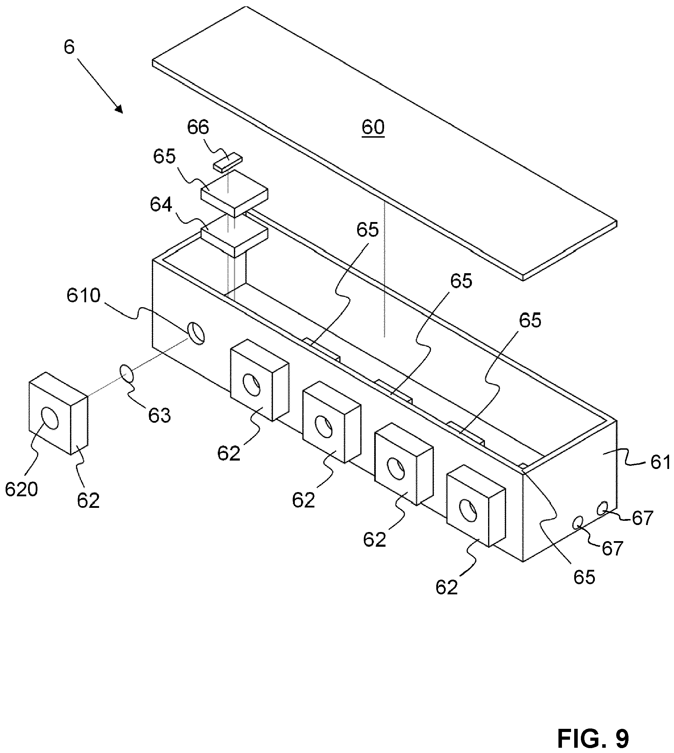

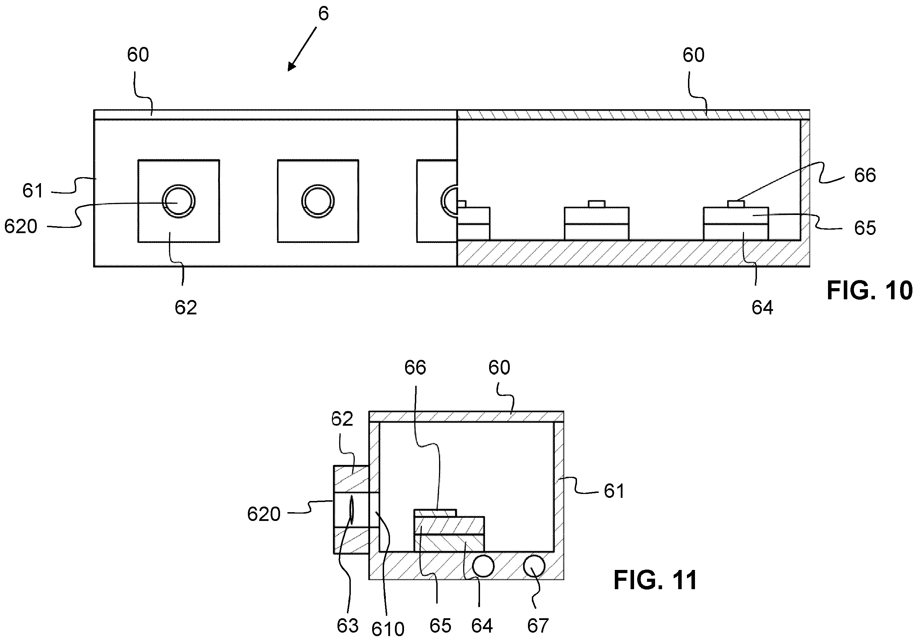

Figures 9 to 11 show the laser housing 6 of the inventive gas analyser apparatus. The housing comprises a main body 61 and a lid 60. The main body 61 has preferably the shape of a square-like box or a similar box with a similar shape which is open at the top. It is preferably milled or cut from one single block. The main body 61 and/or the lid 60 are preferably made of aluminium. The housing 6 is preferably hermetically sealed using a sealing element, such as a circumferential O-ring, between lid 60 and main body 61. The sealing element is not shown in the figures. The lid 60 is preferably removably fixed to the main body 61.

The main body 61 comprises cooling ports 67 for connection with the coolant lines 912, 913. The bottom of the main body 61 as shaped to circulate a cooling fluid flowing through these ports 67.

On long side wall of the main body 61 comprises windows 610 in the form of through-holes. A lens mount 62 is fixed to the main body 61 in front of each window 610. Preferably it is screwed or otherwise removably mounted to the main body 61. Each lens mount 62 holds at least one lens 63 within an own hole 620, the lens being preferably adjustable with some screws or with any other kinds of known adjustment means.

In the interior of the main body 61, there are at least two slots or places, here five slots, to receive laser. There can also be another number of slots. On each slot or place, a temperature control element 64, preferably a Peltier element, is arranged on the bottom of the main body 61.

On each temperature control element 64, a heat sink 65, such as a copper plate, is arranged. The heat sink 65 carries the laser 66, preferably a QCL (Quantum Cascade Laser), an ICL (Interband Cascade Laser) or TDLs (tuneable diode lasers).

Each laser 66 is driven separately. The wires connecting the lasers 66 with electronic elements and the power supply and therefore leaving the laser housing 6, are not shown. They preferably pass either through the lid 60 or the back wall of the main body 61. The corresponding holes are preferably sealed, for example, the connectors are glued to the housing.

The inventive gas analyser apparatus preferably uses a startup-algorithm with pre-defined temperatures for the laser, where the lasers are preferably known to be close to the exact operating wavelength. Once the lasers reach that temperature, they are turned on and the spectrum is recorded.

If the laser emission frequency drifts away from the one in the last run it might also change the laser emission intensity (baseline). As the newly recorded spectrum contains some absorption lines it hinders from measuring the baseline directly. Withing the typical laser drifts though the shape of the emission intensity stays almost the same. This helps to find the new baseline by scaling the baseline from the previous run such that it "touches" the new measured spectrum from above. This allows to recalculate the experimental spectrum into absorbance. Then a cross correlation of the derivative of the found absorbance with the derivative of the HITRAN-simulated absorbance determines the frequency shift between the previously stored absorption line position (fitting parameter) and the new line position. The absorption line position of the fitting function is adjusted to start fitting the absorption line. The new starting fitting parameters are therefore found and the line of interest is successfully fitted.

Once the absorption line is fitted, the software gives a feedback to regulate the laser heatsink temperature to move the absorption line position to the original.

This procedure allows a robust automatic startup and a reproducible (and therefore accurate) fitting result. It also allows to automatically "find" absorption lines after a fit was lost, e.g. due to a too low/high signal or instrument drifts.

The procedure is performed sequentially (within seconds) for all lasers in the instrument.

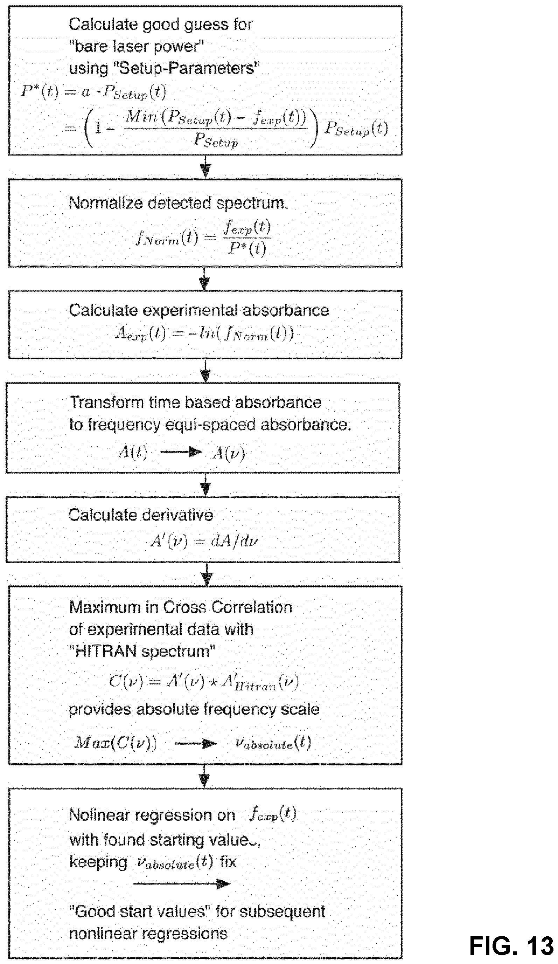

Figure 13 shows a flow diagram of the inventive start-algorithm, which is explained in more detail in the summary part of this text. Figures 12a to 12f illustrate graphs showing the steps in the start-up slgorithm, which steps are mentioned in more detail in the summary part of this text.

The inventive gas analyser enables a compact device which provides accurate and precise measurements of a multiple of gases and which is quite easy to handle. The inventive start-up method is one element, which improves handling and accuracy.

## LIST OF REFERENCE SIGNS

| | | | |
|---|---|---|---|
| 1 | analyser housing | 431 | main body |
| 10 | display | 432 | central through-opening |
| 11 | fan | 433 | fixation opening |
| 12 | dividing wall | 434 | indentation |
| 13 | electronic interface | 44 | third mounting ring |
| 140 | first coolant port | 440 | middle ring |
| 141 | second coolant port | 441 | outer fixation through opening |
| 150 | first port | | |
| 151 | second port | 442 | inner fixation opening |
| 152 | third port | 443 | micro-screw through-opening |
| | | 444 | outer fixation opening |
| 2 | multipass cell | 445 | central through-opening |

(continued)

| | | | |
|---|---|---|---|
| 20 | main tube | 446 | sealing ring |
| 21 | gas inlet | 447 | sealing ring |
| 22 | gas outlet | 448 | inner flansch |
| 23 | back face | 450 | first spring |
| 3 | front mirror holder | 451 | inner ring |
| | | 452 | second spring |
| 4 | back mirror holder | 453 | central through-opening |
| 40 | first mounting ring | 454 | fixation through-opening |
| 400 | fixation opening ??? | 455 | slit |
| 401 | central through-opening | | |
| 41 | second mounting ring | 46 | lever arm body |
| 410 | fixation through-opening | 460 | lever arm |
| 411 | central through-opening | 461 | front face |
| 412 | step | 463 | fixation hole |
| 413 | sealing ring | 470 | first micro-screw |
| 42 | back mirror | 471 | second micro-screw |
| 420 | stem | 472 | third micro-screw |
| 421 | central opening | | |
| 43 | guiding element | 48 | micro-screw holder |
| 430 | flange | 480 | main body |
| 481 | flange | 67 | cooling port |
| 482 | fixation through-opening | | |
| 483 | central micro-screw opening | 7 | deflection mirror |
| 484 | lateral micro-screw opening | | |
| 485 | indentation | 80 | data acquisition device (DAQ) |
| 486 | recess | | |
| | | 81 | data processing device |
| 49 | end piece | 82 | driving electronics |
| 490 | flange | | |
| 491 | fixation through-opening | 90 | suction pump |
| 492 | main body | 901 | external suction line |
| 493 | sensor port | 902 | inlet line |
| | | 903 | valve |
| 5 | photodetector | 904 | first gas line |
| | | 905 | flushing line |
| 6 | laser housing | 906 | second gas line |
| 60 | lid | | |
| 61 | main body | 91 | cooling device |
| 610 | window | 910 | first external coolant line |
| 62 | lens mount | 911 | second external coolant line |
| 620 | hole | 912 | first internal coolant line |
| 63 | lens | 913 | second internal coolant line |
| 64 | temperatur control element | | |
| 65 | heat sink | L | light path |
| 66 | laser | | |

## Claims

1. A gas analyser apparatus using laser absorption spectroscopy wherein the gas analyser comprises a multipass cell with a cell body (2), a front mirror holder (3) and a back mirror holder (3), wherein the cell body (2) has a main tube (20) with an interior space for receiving gas to be analysed and wherein the cell body (2) has an gas inlet (21) for leading the gas into this interior space and an gas outlet (22) for leading the gas out of this interior space, wherein the main tube (20) is made of glass, **characterized in that** the gas inlet (21) and the gas outlet (22) are made of glass.

2. The gas analyser apparatus according to claim 1 wherein the cell body (2) is completely made of glass.

3. The gas analyser apparatus according to one of claims 1 or 2 wherein the gas inlet (21) and/or the gas outlet (22) are shaped as tubes.

4. The gas analyser apparatus according to one of claims 1 to 3 wherein the gas inlet (21) and/or the gas outlet (22) are attached to the main tube (202) by welding.

5. The gas analyser apparatus according to one of claims 1 to 4 wherein the gas inlet (21) and/or the gas outlet (22) are arranged at a distance to the front mirror holder (3) and the back mirror holder (4).

6. The gas analyser apparatus according to one of claims 1 to 5 wherein the back mirror holder (4) comprises a back mirror (42) and adjustment means (460, 470, 471, 472) for adjusting the back mirror (42) wherein the adjustment means comprises a lever arm (460) which is rotatable around a longitudinal middle axis of the multipass cell (2) by movement of a micro-screw (471) which can be moved in a direction perpendicular to the longitudinal middle axis of the multipass cell (2).

7. The gas analyser apparatus according to 6 wherein the lever arm (460) is arranged on a lever arm body (46) which lever arm body (46) is translatable in a direction parallel to the longitudinal middle axis of the multipass cell (2) independently of the rotatable movement by movement of a further micro-screw (472) which can be moved in a direction parallel to the longitudinal middle axis of the multipass cell (2).

8. The gas analyser apparatus according to one of claims 6 or 7 wherein the back mirror holder (4) comprises a mounting ring (44), a guiding element (43), a stem (420) attached to the back mirror (42), a lever arm body (46) with a lever arm (460) extending radially from the lever arm body (46), first micro-screws (470), a second micro-screw (471) and a third micro-screw (471),
wherein the mounting ring (44) receives the guiding element (43),
wherein the guiding element (43) receives the stem (420), the guiding element (43) having a flange (430) comprising indentations (434) for being contacted by one of the first micro-screws (470) each,
wherein the lever arm body (46) has a blind hole receiving the stem (420) of the mirror (42),
wherein the second micro-screw (471) acts on the lever arm (460) to rotate the lever arm (460) around the longitudinal middle axis and
wherein the third micro-screw (472) acts on a front face (461) of the lever arm body (46) in order to move the stem (420) of the mirror (42) along the longitudinal middle axis.

9. The gas analyser apparatus according to claim 8 wherein the back mirror holder (4) comprises a micro-screw holder (48) for holding the second and the third micro-screw (471, 472), for receiving the lever arm body (46) and for being attached together with the lever arm body (46) to the mounting ring (44), wherein the micro-screw holder (4) has a recess (486) for allowing rotational movement of the lever arm (460).

10. The gas analyser apparatus according to one of claims 1 to 9 wherein the gas analyser apparatus comprises at least two lasers (66), preferably up to five, six or seven lasers (66), and a laser housing (6), wherein the at least two lasers (66) are mounted in the housing (6) and wherein the housing (6) comprises a monolithic main body (61) defining an interior space for receiving the lasers and a lid (60) for sealingly closing the main body (61).

11. The gas analyser apparatus according to claim 10 wherein the laser housing (6) comprises a side wall with a through opening (610) for each laser (66) and wherein a lens mount (62) with at least one lens (63) is mounted on the laser housing (6) at each of these through openings (610).

12. The gas analyser apparatus according to one of claims 1 to 11 wherein the gas analyser comprises an astigmatic Herriott type multipass cell or a nonastigmatic Herriott type cell.

13. A method for starting-up a gas analyser apparatus using laser absorption spectroscopy, especially a gas analyser apparatus based on direct laser absorption spectroscopy, the method comprising the following steps

- calculating a good guess for a "bare power laser" using set-up parameters,
- normalizing a detected spectrum,
- calculating an experimental absorbance,
- transforming a time based absorbance to a equi spaced, frequency based absorbance,
- calculating a derivative of frequency equi-spaced absorbance,
- finding a maximum in cross corelation of experimental data with a "HITRAN" spectrum or based on a custom made database in order to find the absolute frequencies,
- perfoming a nonlinear regression on the detected spectrum with the found absolute frequencies as the starting parameters, thereby obtaining new start values for subsequent nonlinear regressions.

14. A gas analyser apparatus using laser absorption spectroscopy wherein the gas analyser comprises an optical sample cell with a cell body (2), a front mirror holder (3) and a back mirror holder (4), wherein the back mirror holder (4) comprises a back mirror (42) and adjustment means (460, 470, 471, 472) for adjusting the back mirror (42), **characterized in that** the adjustment means comprises a lever arm (460) which is rotatable around a longitudinal middle axis of the optical sample cell (2) by movement of a micro-screw (472) which can be moved in a direction perpendicular to the longitudinal middle axis of the optical sample cell (2).

15. A gas analyser apparatus using laser absorption spectroscopy wherein the gas analyser comprises at least two lasers (66), preferably up to five, six or seven lasers (66), and a laser housing (6), wherein the at least two lasers (66) are mounted in the laser housing (6) and wherein the laser housing (6) comprises a monolithic main body (61) defining an interior space for receiving the lasers (66) and a lid (60) for sealingly closing the main body (61), wherein the laser housing (6) is arranged within an outer housing (1) of the gas analyser apparatus.

1

10

11

FIG. 1

FIG. 2

3

21

2

1

**FIG. 3**

20

22

4

41

493

40

41

3

21

2

22

4

20

**FIG. 4**

FIG. 5

FIG. 6

FIG. 7

FIG. 8

FIG. 9

FIG. 10

FIG. 11

FIG. 12a

FIG. 12b

FIG. 12c

Step 4

**FIG. 12d**

Step 6, 7

**FIG. 12e**

Step 8

**FIG. 12f**

Calculate good guess for
"bare laser power"
using "Setup-Parameters"

$$P^*(t) = a \cdot P_{Setup}(t)$$
$$= \left(1 - \frac{Min\left(P_{Setup}(t) - f_{exp}(t)\right)}{P_{Setup}}\right) P_{Setup}(t)$$

Normalize detected spectrum.
$$f_{Norm}(t) = \frac{f_{exp}(t)}{P^*(t)}$$

Calculate experimental absorbance
$$A_{exp}(t) = -ln(f_{Norm}(t))$$

Transform time based absorbance
to frequency equi-spaced absorbance.
$$A(t) \longrightarrow A(\nu)$$

Calculate derivative
$$A'(\nu) = dA/d\nu$$

Maximum in Cross Correlation
of experimental data with
"HITRAN spectrum"
$$C(\nu) = A'(\nu) \star A'_{Hitran}(\nu)$$
provides absolute frequency scale
$$Max(C(\nu)) \longrightarrow \nu_{absolute}(t)$$

Nolinear regression on $f_{exp}(t)$
with found starting value,
keeping $\nu_{absolute}(t)$ fix

"Good start values" for subsequent
nonlinear regressions

FIG. 13

Europäisches
Patentamt

European
Patent Office

Office européen
des brevets

# EUROPEAN SEARCH REPORT

Application Number

EP 20 19 8427

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MCMANUS J B ET AL: "Compact Quantum Cascade Laser Instrument for Rapid, High Sensitivity Measurements of Trace Gases in Air", SENSORS, 2007 IEEE, IEEE, PI, 28 October 2007 (2007-10-28), pages 1341-1344, XP031221319, ISBN: 978-1-4244-1261-7 * abstract * * figures 1, 2 * * section "II. INTSTRUMENT DESIGN" * | 1,3,5 | INV. G01N21/03 ADD. G01N21/05 G01N21/3504 G01N21/39 |
| X | WO 2017/081363 A1 (TEKNOLOGIAN TUTKIMUSKESKUS VTT OY [FI]) 18 May 2017 (2017-05-18) * paragraphs [0031] - [0035], [0044] * * figures 1a, 1b * | 1,2,5 | |
| X | US 4 914 297 A (WIEBOLDT RICHARD C [US] ET AL) 3 April 1990 (1990-04-03) * figures 1, 2 * * column 3, line 44 - column 5, line 68 * | 1-5 | |
| X | MCMANUS J B ET AL: "Pulsed quantum cascade laser instrument with compact design for rapid, high sensitivity measurements of trace gases in air", APPLIED PHYSICS B ; LASERS AND OPTICS, SPRINGER, BERLIN, DE, vol. 92, no. 3, 12 August 2008 (2008-08-12), pages 387-392, XP019627126, ISSN: 1432-0649 * abstract * * figures 1, 2 * * table 1 * * the corresponding description * | 1,3,5 | TECHNICAL FIELDS SEARCHED (IPC) G01N |

-/--

~~The present search report has been drawn up for all claims~~

| Place of search | Date of completion of the search | Examiner |
|---|---|---|
| Munich | 11 March 2021 | Sauerer, Christof |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 20 19 8427

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 807 750 A (BAUM MARC M [US] ET AL) 15 September 1998 (1998-09-15) * figure 2 * * column 7, line 49 - column 8, line 4 * ----- | 1-5 | |

TECHNICAL FIELDS
SEARCHED        (IPC)

~~The present search report has been drawn up for all claims~~

| Place of search | Date of completion of the search | Examiner |
|---|---|---|
| Munich | 11 March 2021 | Sauerer, Christof |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

Europäisches
Patentamt

European
Patent Office

Office européen
des brevets

**Application Number**

EP 20 19 8427

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-5

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

## LACK OF UNITY OF INVENTION
### SHEET B

The Search Division considers that the present European patent application does not comply with the requirements of unity of invention and relates to several inventions or groups of inventions, namely:

1. claims: 1-5

   A gas analyser apparatus using laser absorption spectroscopy wherein the gas analyser comprises a multipass cell with a cell body, a front mirror holder and a back mirror holder, wherein the cell body has a main tube with an interior space for receiving gas to be analysed and wherein the cell body has an gas inlet for leading the gas into this interior space and an gas outlet for leading the gas out of this interior space, wherein the main tube is made of glass, and wherein that the gas inlet and the gas outlet are made of glass,
   wherein details of the material and arrangement of the cell body and gas inlet and outlets are further specified.
   ---

2. claims: 6-9, 14

   A gas analyser apparatus using laser absorption spectroscopy wherein the gas analyser comprises an optical sample cell with a cell body, a front mirror holder and a back mirror holder, wherein the back mirror holder comprises a back mirror and adjustment means for adjusting the back mirror, characterized in that the adjustment means comprises a lever arm which is rotatable around a longitudinal middle axis of the optical sample cell by movement of a micro-screw which can be moved in a direction perpendicular to the longitudinal middle axis of the optical sample cell.
   OR
   A gas analyser apparatus using laser absorption spectroscopy wherein the gas analyser comprises a multipass cell with a cell body, a front mirror holder and a back mirror holder, wherein the cell body has a main tube with an interior space for receiving gas to be analysed and wherein the cell body has an gas inlet for leading the gas into this interior space and an gas outlet for leading the gas out of this interior space, wherein the main tube is made of glass, and wherein that the gas inlet and the gas outlet are made of glass,
   wherein details of the back mirror holder are further specified.
   ---

3. claims: 10-12, 15

   A gas analyser apparatus using laser absorption spectroscopy wherein the gas analyser comprises at least two lasers, preferably up to five, six or seven lasers, and a laser housing, wherein the at least two lasers are mounted in the laser housing and wherein the laser housing comprises a monolithic main body defining an interior space for

Europäisches
Patentamt

European
Patent Office

Office européen
des brevets

**LACK OF UNITY OF INVENTION
SHEET B**

Application Number

EP 20 19 8427

The Search Division considers that the present European patent application does not comply with the requirements of unity of invention and relates to several inventions or groups of inventions, namely:

receiving the lasers and a lid for sealingly closing the main body, wherein the laser housing is arranged within an outer housing of the gas analyser apparatus.
OR
A gas analyser apparatus using laser absorption spectroscopy wherein the gas analyser comprises a multipass cell with a cell body, a front mirror holder and a back mirror holder, wherein the cell body has a main tube with an interior space for receiving gas to be analysed and wherein the cell body has an gas inlet for leading the gas into this interior space and an gas outlet for leading the gas out of this interior space, wherein the main tube is made of glass, and wherein that the gas inlet and the gas outlet are made of glass,
wherein the gas analyser apparatus comprises a plurality of lasers and a laser housing.
---

4. claim: 13

A method for starting-up a gas analyser apparatus using laser absorption spectroscopy, especially a gas analyser apparatus based on direct laser absorption spectroscopy, the method comprising the following steps
- calculating a good guess for a "bare power laser" using set-up parameters,
- normalizing a detected spectrum,
- calculating an experimental absorbance,
- transforming a time based absorbance to a equi spaced, frequency based absorbance,
- calculating a derivative of frequency equi-spaced absorbance,
- finding a maximum in cross corelation of experimental data with a "HITRAN" spectrum or based on a custom made database in order to find the absolute frequencies,
- perfoming a nonlinear regression on the detected spectrum with the found absolute frequencies as the starting parameters,thereby obtaining new start values for subsequent nonlinear regressions.
---

## ANNEX TO THE EUROPEAN SEARCH REPORT
## ON EUROPEAN PATENT APPLICATION NO.

EP 20 19 8427

This annex lists the patent family members relating to the patent documents cited in the above-mentioned European search report.
The members are as contained in the European Patent Office EDP file on
The European Patent Office is in no way liable for these particulars which are merely given for the purpose of information.

11-03-2021

| Patent document cited in search report | | Publication date | Patent family member(s) | | Publication date |
|---|---|---|---|---|---|
| WO 2017081363 | A1 | 18-05-2017 | EP<br>US<br>WO | 3380824 A1<br>2018372616 A1<br>2017081363 A1 | 03-10-2018<br>27-12-2018<br>18-05-2017 |
| US 4914297 | A | 03-04-1990 | NONE | | |
| US 5807750 | A | 15-09-1998 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2940808 B1

**Non-patent literature cited in the description**

- **C. LIU et al.** Laser driving and data processing concept for mobile trace gas sensing: Design and implementation. *RSI,* 2018, vol. 89, 065107
- **M. HUNDT et al.** Multi-species trace gas sensing with dual-wavelength QCLs. *APB,* 2018, vol. 124, 108
- **O. ASEEV et al.** High-precision ethanol measurement by mid-IR laser absorption spectroscopy for metrological applications. *Optics Express,* 2019, vol. 27, 5314-5325
- Applications of quantum cascade lasers to trace gas sensing. *Appl. Phys. B,* 2008, vol. 90, 165-176
- **M. HUNDT et al.** Mid-IR spectrometer for mobile, real-time urban NO2 measurements. *AMT,* 2018, vol. 11, 2669-2681
- Journal of Quantitative Spectrocopy and Radiative Transfer. Elsevier, December 2017, vol. 203, 3-69